# EUROPÄISCHE PATENTSCHRIFT

(11) **EP 1 309 542 B1**
(45) Veröffentlichungstag und Bekanntmachung des Hinweises auf die Patenterteilung: **31.05.2006**
(21) Anmeldenummer: 01971836.0
(22) Anmeldetag: 01.08.2001
(51) Int. Cl.: C07C 253/32

(54) **VERFAHREN ZUR HERSTELLUNG VON HYDROLYSESTABILEN AMMONIUMNITRILEN**
METHOD FOR PRODUCING HYDROLYSIS-STABLE AMMONIUM NITRILES
PROCEDE DE PRODUCTION DE NITRILES D'AMMONIUM STABLES A L'HYDROLYSE

(30) Priorität: 04.08.2000 DE 10038086
(43) Veröffentlichungstag der Anmeldung: 14.05.2003
(73) Patentinhaber: Clariant Produkte (Deutschland) GmbH, 65929 Frankfurt am Main (DE)
(72) Erfinder: SCHREIBER, Manfred, 65929 Frankfurt (DE); BORCHERS, Georg, 61231 Bad Nauheim (DE); MOGCK, Oliver, 84508 Burgkirchen (DE); WEINELT, Frank, 84508 Burgkirchen (DE)
(86) Internationale Anmeldenummer: PCT/EP2001/008883
(87) Internationale Veröffentlichungsnummer: WO 2002/012175

(56) Entgegenhaltungen:
- EP-A- 0 464 880

## Beschreibung

Vorliegende Erfindung betrifft ein Verfahren zur Herstellung von hydrolysestabilen Ammoniumnitrilen durch Umsetzung von Ammoniumnitrilen mit Sulfonaten.

Bleichaktivatoren sind wichtige Bestandteile in Kompaktwaschmitteln, Fleckensalzen und Maschinengeschirrspülmitteln. Sie ermöglichen bereits bei 40-60°C ein der Kochwäsche vergleichbares Bleichergebnis, indem sie mit Wasserstoffperoxidspendern (meist Perborate, Percarbonate, Persilicate und Perphosphate) unter Freisetzung von Peroxysäuren reagieren.

Viele Substanzen sind nach dem Stand der Technik als Bleichaktivatoren bekannt. Gewöhnlich handelt es sich dabei um reaktive organische Verbindungen mit einer O-Acyl- oder N-Acyl-Gruppe, die in alkalischer Lösung zusammen mit einer Quelle für Wasserstoffperoxid die entsprechenden Peroxysäuren bilden.

Repräsentative Beispiele für Bleichaktivatoren sind etwa N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglucoluril (TAGU), Tetraacetylcyansäure (TACA), Di-N-acetyldimethylglyoxin (ADMG) und 1-Phenyl-3-acetylhydantoin (PAH).

Ammoniumnitrile der Formel 1 bilden eine besondere Klasse kationischer Bleichaktivatoren. Verbindungen dieser Art und deren Verwendung als Bleichaktivator in Bleichmitteln sind in EP-A-0 303 520, EP-A-0 464 880, EP-A-0 458 396, EP-A-0 897 974 und EP-A-0 790 244 beschrieben. 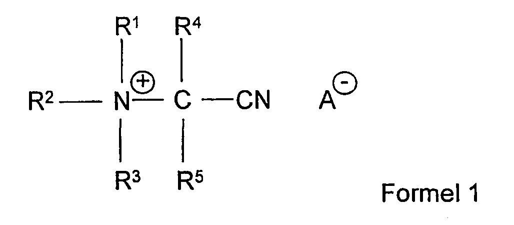 worin R¹, R², R³ gleich oder verschieden sind, und für lineare oder verzweigte C₁-C₂₄-Alkylgruppen, C₂-C₂₄-Alkenylgruppen oder für C₁-C₄-Alkoxy-C₁-C₄₋Alkylgruppen, substituiert oder unsubstituiertes Benzyl stehen, oder worin R¹ und R² zusammen mit dem Stickstoffatom, an das sie gebunden sind, einen Ring mit 4 bis 6 C-Atomen bilden, der mit C₁-C₅-Alkyl, C₁-C₅-Alkoxy, C₁-C₅-Alkanoyl, Phenyl, Amino, Ammonium, Cyano, Cyanamino, Chlor oder Brom substituiert sein kann und zusätzlich zum Stickstoffatom anstelle von Kohlenstoffatomen ein oder zwei Sauerstoff- oder Stickstoffatome, eine Gruppe N-R⁶ oder eine Gruppe R³-N-R⁶ enthalten kann, worin R⁶ Wasserstoff, C₁-C₅-Alkyl, C₂-C₅-Alkenyl, C₂-C₅-Alkinyl, Phenyl, C₇-C₉-Aralkyl, C₅-C₇-Cycloalkyl, C₁-C₆-Alkanoyl, Cyanomethyl oder Cyan ist, R⁴ und R⁵ Wasserstoff, C₁-C₄-Alkyl, C₁-C₄-Alkenyl, C₁-C₄-Alkoxy-C₁-C₄-alkyl, Phenyl oder C₁-C₃-Alkylphenyl, vorzugsweise Wasserstoff, Methyl oder Phenyl sind, wobei insbesondere R⁴ Wasserstoff bedeutet, wenn R⁵ keinen Wasserstoff bedeutet, und A für ein Anion, beispielsweise Chlorid, Bromid, lodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methylsulfat, Phosphonat, Methylphosphonat, Methandisulfonat, Methylsulfonat, Ethansulfonat steht.

Die Synthese von quarternierten Ammoniumnitrilen erfolgt in bekannter Weise durch Umsetzung eines sekundären Amins mit Natriumcyanid und Formaldehyd und anschließender Methylierung mit beispielsweise Methylchlorid, Benzylchlorid oder Dimethylsulfat oder durch Umsetzung von sekundären Aminen mit Chloracetonitril.

Für den Einsatz der Ammoniumnitrile als Bleichaktivator in Wasch- und Reinigungsmitteln ist die Hygroskopizität der Ammoniumnitrile und die Hydrolyseempfindlichkeit in Gegenwart alkalischer Waschmittelbestandteile und eine damit verbundene entsprechend geringe Lagerstabilität von großem Nachteil. In EP-A-0 464 880 wird beschrieben, dass die Hygroskopizität von Ammoniumnitrilen durch Art und Größe der Anionen beeinflusst wird. Ammoniumnitrile mit Alkan- oder Paraffinsulfonat, Arylsulfonat, primärem Alkoholsulfat, oder Fettsäurealkylcarboxylat als Gegenion sind im Vergleich zu Ammoniumnitrilen mit konventionellen Anionen, wie beispielsweise Chlorid, Nitrat oder Methosulfat stabiler. Die Schrift lehrt, dass Ammoniumnitrile mit den Gegenionen RSO₃⁻, RSO₄⁻ oder RCO₂⁻ durch Anionenaustausch aus Ammoniumnitril mit Chlorid oder Methylsulfat als Gegenion erhalten werden. Dabei wird das Ammoniumnitril-Chlorid bzw. -Methylsulfat in Methanol/Isopropylalkohol gelöst und durch Zugabe des Natriumsalzes eines Sulfonates, Sulfates bzw. Carboxylates das entsprechende Ammoniumnitril zur Fällung gebracht. Alternativ wird ein Trockenvermischen des Ammoniumnitrils mit konventionellen Anionen mit einem Natriumsalz eines Sulfonates, Sulfates bzw. Carboxylates vorgeschlagen, wobei kein wirklicher Annionenaustausch erfolgt und das resultierende Produkt verklebt und nur mäßig stabil ist.

Der Anionenaustausch durch Fällungsreaktion in polaren organischen Lösungsmitteln wie z.B. Methanol und Isopropanol ist aus ökologischen und ökonomischen Gründen nachteilig; die Abtrennung der Lösungsmittel aus dem Fällungsprodukt aufwendig.

Überraschenderweise wurde gefunden, dass der zur Verminderung der Hydrolyseempfindlichkeit und der Hygroskopizität von Ammoniumnitrilen erforderliche Anionenaustausch konventioneller Anionen, beispielsweise Chlorid oder Methylsulfat gegen Alkan- oder Paraffinsulfonat, Arylsulfonat, primärem Alkoholsulfat, beispielsweise Laurylsulfat oder Fettsäurealkylcarboxylat, in einfacher Weise in Wasser erfolgen kann.

Gegenstand der Erfindung ist ein Verfahren zur Herstellung von hydrolysestabilen Ammoniumnitrilen durch Umsetzung einer Verbindung der Formel 1. 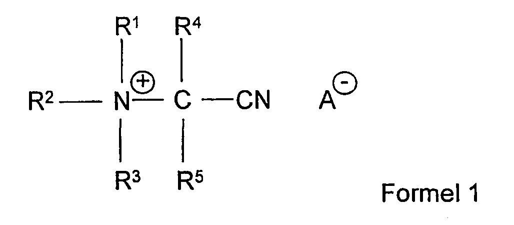 worin R¹, R², R³ gleich oder verschieden sind, und für lineare oder verzweigte C₁-C₂₄-Alkylgruppen, C₂-C₂₄-Alkenylgruppen oder für C₁-C₄-Alkoxy-C₁-C₄₋Alkylgruppen, substituiert oder unsubstituiertes Benzyl stehen, oder worin R¹ und R² zusammen mit dem Stickstoffatom, an das sie gebunden sind, einen Ring mit 4 bis 6 C-Atomen bilden, der mit C₁-C₅-Alkyl, C₁-C₅-Alkoxy, C₁-C₅-Alkanoyl, Phenyl, Amino, Ammonium, Cyano, Cyanamino, Chlor oder Brom substituiert sein kann und zusätzlich zum Stickstoffatom anstelle von Kohlenstoffatomen ein oder zwei Sauerstoff- oder Stickstoffatome, eine Gruppe N-R⁶ oder eine Gruppe R³-N-R⁶ enthalten kann, worin R⁶ Wasserstoff, C₁-C₅-Alkyl, C₂-C₅-Alkenyl, C₂-C₅-Alkinyl, Phenyl, C₇-C₉-Aralkyl, C₅-C₇-Cycloalkyl, C₁-C₆-Alkanoyl, Cyanomethyl oder Cyan ist, R⁴ und R⁵ Wasserstoff, C₁-C₄-Alkyl, C₁-C₄-Alkenyl, C₁-C₄-Alkoxy-C₁-C₄-alkyl, Phenyl oder C₁-C₃-Alkylphenyl, vorzugsweise Wasserstoff, Methyl oder Phenyl sind, wobei insbesondere R⁴ Wasserstoff bedeutet, wenn R⁵ keinen Wasserstoff bedeutet, und A für ein Anion steht, mit einer Verbindung der Formeln RSO₃X, R¹SO₄X oder R²CO₂X, wobei R, R¹ und R² C₄-C₂₀-, vorzugsweise C₁₀-C₁₈-Alkyl, oder substituiertes Aryl, vorzugsweise C₁-C₁₈-Alkylphenyl, und X ein Alkali-, Ammonium- oder Wasserstofflon bedeutet, wobei die Nitrile der Formel 1 in Wasser bei 50 bis 100°C, bis zur Sättigung gelöst, mit einer im gleichen Temperaturbereich gesättigten wässrigen Lösung der Verbindungen der Formeln RSO₃X, R¹SO₄X oder R²CO₂X vermischt und die Ammoniumnitril-sulfonate, - sulfate bzw. -carboxylate nach Abkühlen des Gemischs abgetrennt werden.

Als Verbindungen der Formeln RSO₃X, R¹SO₄X und R²CO₂X kommen alle allgemein bekannten und gebräuchlichen Alkansulfonate, Paraffinsulfonate, substituierte Arylsulfonate, Alkylsulfate und Fettsäurealkylcarboxylate in Frage.

Das erfindungsgemäße Verfahren wird vorzugsweise in der Weise durchgeführt, dass die Nitrile der Formel 1 in Wasser bei 60 bis 90°C, insbesondere 70 bis 85°C bis zur Sättigung gelöst und mit einer im gleichen Temperaturbereich gesättigten wässrigen Lösung der Verbindungen der Formeln RSO₃X, R¹SO₄X oder R²CO₂X, beispielsweise Cumolsulfonat, Laurylsulfat oder Fettsäurealkylcarboxylaten vermischt werden. Die im Vergleich zu den Ausgangssalzen schwerer löslichen Ammoniumnitril-sulfonate, -sulfate bzw. -carboxylate werden durch Fällung als weiße Feststoffe erhalten. Dazu wird die Gemischlösung abgekühlt.

Das Molverhältnis der Verbindungen der Formeln RSO₃(X), RSO₄(X) bzw. RCO₂(X) und der Nitrile der Formel 1 liegt im Bereich von 1 zu 2 bis 5 zu 1 bevorzugt 1 zu 1 bis 4 zu 1.

In einer bevorzugten Ausführungsform wird eine 40 %ige, wässrige, klare Lösung von Natriumcumolsulfonat und/oder Natriumlaurylsulfat mit einer 50 bis 80 %igen wässrigen, klaren Lösung von Cyanomethyltrimethylammoniummethylsulfat und/oder -chlorid bei den zuvor angegebenen Temperaturen vermischt, wobei das Molverhältnis Natriumcumolsulfonat bzw. Natriumlaurylsulfat- und Ammoniumnitril-Chlorid bzw. -methosulfat 1 zu 1 bis 2 zu 1 beträgt.
Die im Vergleich zu den Ausgangssalzen schwerer löslichen Ammoniumnitril-sulfonate, -sulfate bzw. -carboxylate kristallisieren bei Temperaturen im Bereich von 40°C bis 0°C, bevorzugt bei ca. 20°C aus und können abfiltriert, gegebenenfalls umkristallisiert und getrocknet werden.

In einer weiteren erfindungsgemäßen Verfahrensweise werden Ammoniumnitrile mit konventionellen Anionen gemäß Formel 1 in Wasser bei 50 bis 100°C, bevorzugt 60 bis 90°C, insbesondere 70 bis 85°C gelöst und mit einer wässrigen Lösung, die auf eine in diesem Temperaturbereich liegende Temperatur erwärmt ist, von Verbindungen der Formeln RSO₃X, R¹SO₄X oder R²CO₂X, beispielsweise Cumolsulfonat, Laurylsulfat, C_{12/18}-Alkylsulfat oder Fettsäurealkylcarboxylaten homogen vermischt. Die Lösung wird nicht zur Kristallisation gebracht, sondern im zuvor angegebenen Temperaturbereich einer Sprühtrocknung unterzogen, wobei die Gasaustrittstemperatur im Bereich von 100°C bis 150°C liegt. Der so erhaltene weiße, pulverige Feststoff setzt sich zusammen aus Nitrilsalzen mit dem Anion gemäß den Formeln RSO₃^{⊖}, R¹SO₃^{⊖} oder R²COO^{⊖}, den Ausgangsverbindungen und Alkali/Ammoniumsalzen mit dem Anion A, wobei Anionen und Kationen statistisch verteilt vorliegen.
In einer weiteren bevorzugten Ausführungsform zur Minimierung der Hydrolyseempfindlichkeit, der Hygroskopizität, sowie des Verflüssigens von Ammoniumnitrilen werden Ammoniumnitrile mit konventionellen Anionen gemäß Formel 1 in Wasser bei 50 bis 100°C, bevorzugt 60 bis 90°C, insbesondere 70 bis 85°C gelöst und mit einer im gleichen Temperaturbereich gesättigten, wässrigen Lösung von Verbindungen der Formeln RSO₃X, R¹SO₄X oder R²COOX beispielsweise Cumolsulfonat, Laurylsulfat, C_{12/18}-Alkylsulfat, sowie mit einer wässrigen Lösung oder Dispersion eines anorganischen Salzes, beispielsweise Na₂SO₄, die ebenfalls auf die angegebenen Temperaturen erwärmt sein kann, homogen vermischt und bei diesen Temperaturen einer Sprühtrocknung unterzogen, wobei die Gasaustrittstemperatur im Bereich von 100°C bis 150°C liegt. Man erhält einen weißen, pulvrigen Feststoff in Form einer homogenen Mischung mit statistischer Anionenverteilung aus Nitrilsalzen mit den Anionen RSO₃^{⊖}, R¹SO₄^{⊖} oder R²COO^{⊖}, den Ausgangsverbindungen und Alkali/Ammoniumsalzen mit dem Amin A, sowie Alkali-/Ammoniumsulfat, beispielsweise Na₂SO₄.

Der Anionenaustausch kann auch erzielt werden durch trockenes Vermischen der Ammoniumnitrile gemäß der Formel 1, und der Verbindungen der Formeln RSO₃X, R¹SO₄X oder R²COOX im Molverhältnis 1 zu 2 bis 5 zu 1, bevorzugt 1 zu 1 bis 4 zu 1, Zugabe von Wasser und Homogenisieren durch Mischen, Kneten und Dispergieren der pastösen Mischung und anschließende Trocknung.

Durch den oben beschriebenen erfindungsgemäßen Anionenaustausch mit Anionen höherer Molekulargewichte wird eine signifikante Reduzierung der Hygroskopizität und damit eine erhebliche Verbesserung der Lagerstabilität der Ammoniumnitrile erreicht.

Als Bleichaktivator in Wasch- und Reinigungsmitteln besonders bevorzugt sind Ammoniumnitrile gemäß der Formel 2 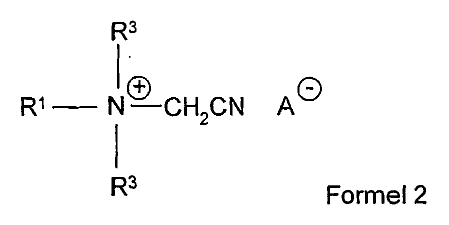 wobei R¹, R² und R³ für eine lineare oder verzweigte, gesättigte oder ungesättigte Alkylgruppe mit 1 bis 24 Kohlenstoffatomen, bzw. 2 bis 24 Kohlenstoffatomen oder substituiert oder unsubstituiertes Benzyl steht und A für ein Anion der Formeln RSO₃^{⊖}, R¹SO₄^{⊖} oder RCOO^{⊖} besonders bevorzugt für Cumolsulfonat und C_{12/18}-alkoholsulfat oder Mischungen der Komponenten steht.

Um eine ausreichende Lagerstabilität zu gewährleisten und die bleichaktivierende Wirkung erst beim Waschvorgang freizusetzen, ist es vorteilhaft, die erfindungsgemäß hergestellten Ammoniumnitrile in granulierter Form einzusetzen. Die erfindungsgemäß hergestellten Ammoniumnitrile können mit oder ohne Zusatz eines Binders verpresst, kompaktiert und schonend bis zu Granulatgrößen von 200 bis 2000 µm zerkleinert werden.

Ebenso geeignet ist eine Aufbaugranulierung im Mischer, beispielsweise im Pflugscharmischer, Ringschichtmischer oder Intensivmischer unter Zusatz eines Binders, insbesondere eines wasserfreien Bindesystems, beispielsweise eines Fettalkoholpolyglykolethers.

In einer weiteren Ausführungsform kann der feuchte Filterkuchen der oben beschriebenen Fällungsreaktion mit oder ohne Zusatz eines Binders einer Formgranulierung durch Matrizen im Extruder, aber auch durch Ringkollerpressen, Kollergänge, gegebenenfalls mit nachgeschaltetem Rondierer, unterzogen werden.
In gleicher Weise kann die getrocknete Festsubstanz der oben beschriebenen Fällungsreaktion bzw. das durch Sprühtrocknung erhaltene trockene Pulver granuliert werden.

Des weiteren kommt eine Wirbelschichtgranulierung einer wässrige Lösung der durch oben beschriebenen Anionenaustausch hergestellten Mischsalze der Ammoniumnitrile in Betracht.

Um Gewebe- und Farbschädigungen zu vermeiden ist es vorteilhaft, die Granulate der erfindungsgemäß hergestellten Ammoniumnitrilsalze mit Coatingsubstanzen zu umhüllen.

Als Coatingmaterial geeignet sind alle filmbildenden Substanzen, wie Wachse, Silikone, Fettsäuren, Seifen, anionische Tenside, nichtionische Tenside, kationische Tenside, sowie anionische und kationische Polymere, z.B. Polyacrylsäuren. Durch Verwendung dieser Coatingmaterialien kann u.a. das Auflöseverhalten verzögert werden, um auf diese Weise auch Wechselwirkungen zwischen dem Bleichaktivator und dem Enzymsystem zu Beginn des Waschprozesses zu unterbinden. Soll das bestimmungsgemäße Granulat in Maschinengeschirrspülmitteln Verwendung finden, eignen sich hierzu vor allem Wachse mit Schmelzpunkten von 40 bis 50°C.

Saure Coatingmaterialien erhöhen die Lagerstabilität der Granulate in percarbonathaltigen, hochalkalischen Formulierungen und unterdrücken Farbschäden durch Spotting. Zusätze eines Farbstoffes sind ebenfalls möglich.

Das Aufbringen der Coatingmaterialien erfolgt in der Regel durch Aufsprühen der geschmolzenen oder in einem Lösemittel gelösten Coatingmaterialien. Gemäß der Erfindung kann das Coatingmaterial in Mengen von 0-20, vorzugsweise von 1-10 Gew.-%, bezogen auf das Gesamtgewicht auf den erfindungsgemäßen Granulatkern aufgebracht werden.

Die erfindungsgemäßen Produkte zeichnen sich durch eine gute Lagerstabilität in pulverförmigen Wasch-, Reinigungs- und Desinfektionsmittelformulierungen aus. Sie sind ideal zum Einsatz in Vollwaschmitteln, Fleckensalzen, Maschinengeschirrspülmitteln, pulverförmigen Allzweckreinigern und Gebissreinigern.

### Beispiele

### Beispiel 1

Es wurde eine 40 %ige wässrige Lösung, die 210 g Cyanomethyltrimethylammoniummethosulfat enthält mit einer 33 %igen wässrigen Lösung, die 222 g Natrium-Cumolsulfonat enthält unter Rühren vermischt. Die Temperaturen der Ausgangslösungen betrugen ca. 30 bis 40°C. Unter Rühren wurde die Gemischlösung auf kleiner 15°C abgekühlt. Dabei wurde ein Feststoff ausgefällt. Durch weitere Abkühlung der Lösung auf Temperaturen auf ca. 3 bis 5°C kann eine zusätzliche Nachfällung des Feststoffes erreicht werden. Der trockene Feststoff wurde auf seine hygroskopischen Eigenschaften vergleichend zu Cyanomethyltrimethylammoniummethosulfat (Nitrilquat A) untersucht.

**Gewichtszunahme bei 80 % rel. Luftfeuchte:**

| Zeit | Nitrilquat A | Fällungsprodukt |
|---|---|---|
| 10 min | 4,9 % | 2,22 % |
| 30 min | 14,5 % | 4,11 % |
| 60 min | 24,6 % | 5,34 % |
| 120 min | 35,1 % | 5,86 % |
| 130 min | 35,9% (flüssig) | 5,75% (festes Pulver) |

### Beispiel 2

10 g einer 58 %igen Lösung von Cyanomethyltrimethylammoniumchlorid in Wasser wurden mit 48 g einer 40 %igen wässrigen Lösung von Natriumcumolsulfonat versetzt und auf 80°C erwärmt. Die klare Lösung wurde in einem Minisprühturm der Fa. Büchi zu einem trockenem Pulver versprüht (Restfeuchtigkeit < 1 Gew.-%). Die Sprühbedingungen sind:

| | |
|---|---|
| Einlasstemperatur: | 220°C |
| Auslasstemperatur: | 111°C |
| Sprühgasmenge zur Düse: | 600 l/h |
| Dosierpumpenleistung: | 9 ml/min |

Das erhaltene Pulver zeigte eine Massezunahme während der Feuchtlagerung (12 h, 78 % rel. Luftfeuchte) von 30 %. Das Pulver bleibt stabil und zeigt keine sichtbare Veränderung der äußeren Form.

### Beispiel 3

13,4 g Cyanomethyltrimethylammoniumchlorid (Nitrilquat B) wurden in 13,4 g Wasser gelöst und mit 44 g Natriumcumolsulfonat als 40 %ige wässrige Lösung versetzt und auf 60 bis 70°C erwärmt. Die erhaltene Lösung wurde unter Vakuum getrocknet. Das erhaltene Pulver wurde bei 80% rel. Luftfeuchte getrocknet und die Gewichtszunahme (in Prozent) beobachtet.

| Zeit | Nitrilquat B | Eindampfprodukt Beispiel 3 |
|---|---|---|
| 44 h | 95% (flüssig) | 25% (fest) |

### Beispiel 4

Aus dem im Beispiel 1 hergestellten nicht-hygroskopischen Fällungsprodukt wurde mittels trockenem Verpressen ein Kompaktat hergestellt. Das Kompaktat wird zum weiteren Schutz mit 10 % Stearinsäure gecoatet. Zur Überprüfung der Lagerstabilität der Granulate wurden diese in einem Anteil von 5 % in eine Waschmittelformulierung eingearbeitet, wobei die Formulierung alle gängigen Waschmittel-Inhaltsstoffe enthält. Die so vorbereiteten Proben wurden bis zu 4 Wochen unter T = 38°C und Umgebungsfeuchte in geschlossenen Gefäßen eingelagert. In regelmäßigen Abständen wird der in den Proben enthaltene Wirkstoff bestimmt.

Folgende Werte der Wirkstoff-Erhaltung (in Prozent) wurden ermittelt:

| Zeit | Nitrilquat A | Fällungsprodukt |
|---|---|---|
| 0 Tage | 100%: | 100% |
| 10 Tage | 83% | 95% |
| 21 Tage | 30 % | 95% |
| 28 Tage | 21% | 85% |

### Beispiel 5

21 g Cyanomethyltrimethylammoniummethosulfat wurden in Wasser gelöst und mit 59,8 Natrium-C_{12/14}-Alkylsulfat als wässrige Lösung versetzt. Dieses Gemisch wurde bei 60 bis 70°C getrocknet. Das erhaltene Pulver wurde bei 80 % rel. Luftfeuchte gelagert. Die Massezunahme betrug nach 44 h 26 %. Das Pulver bleibt stabil und zeigt keine sichtbare Veränderung der äußeren Form.

## Patentansprüche

1. Verfahren zur Herstellung von hydrolysestabilen Ammoniumnitrilen durch Umsetzung einer Verbindung der Formel 1 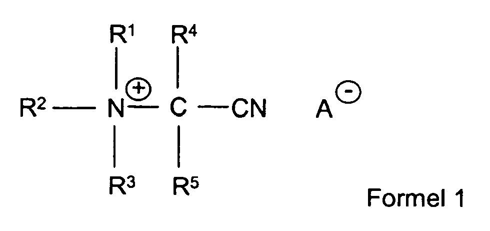 worin R¹, R², R³ gleich oder verschieden sind, und für lineare oder verzweigte C₁-C₂₄-Alkylgruppen, C₂-C₂₄-Alkenylgruppen oder für C₁-C₄-Alkoxy-C₁-C₄₋Alkylgruppen, substituiert oder unsubstituiertes Benzyl stehen, oder worin R¹ und R² zusammen mit dem Stickstoffatom, an das sie gebunden sind, einen Ring mit 4 bis 6 C-Atomen bilden, der mit C₁-C₅-Alkyl, C₁-C₅-Alkoxy, C₁- bis C₅-Alkanoyl, Phenyl, Amino, Ammonium, Cyano, Cyanamino, Chlor oder Brom substituiert sein kann und zusätzlich zum Stickstoffatom anstelle von Kohlenstoffatomen ein oder zwei Sauerstoff- oder Stickstoffatome, eine Gruppe N-R⁶ oder eine Gruppe R³-N-R⁶ enthalten kann, worin R⁶ Wasserstoff, C₁- bis C₅-Alkyl, C₂- bis C₅-Alkenyl, C₂-C₅-Alkinyl, Phenyl, C₇-C₉-Aralkyl, C₅-C₇-Cycloalkyl, C₁-C₆-Alkanoyl, Cyanomethyl oder Cyan ist, R⁴ und R⁵ Wasserstoff, C₁-C₄-Alkyl, C₁-C₄-Alkenyl, C₁-C₄-Alkoxy-C₁-C₄-alkyl, Phenyl oder C₁-C₃-Alkylphenyl, vorzugsweise Wasserstoff, Methyl oder Phenyl sind, wobei insbesondere R⁴ Wasserstoff bedeutet, wenn R⁵ keinen Wasserstoff bedeutet, und A für ein Anion steht, mit einer Verbindung der Formeln RSO₃X, R¹SO₄X oder R²CO₂X, wobei R, R¹ und R² C₄-C₂₀-, vorzugsweise C₁₀-C₁₈-Alkyl oder substituiertes Aryl, vorzugsweise C₁-C₁₈₋Alkylphenyl, und X ein Alkali-, Ammonium- oder Wasserstoff-Ion bedeutet, wobei die Nitrile der Formel 1 in Wasser bei 50 bis 100°C, bis zur Sättigung gelöst, mit einer im gleichen Temperaturbereich gesättigten wässrigen Lösung der Verbindungen der Formeln RSO₃X, R¹SO₄X oder R²CO₂X vermischt und die Ammoniumnitril-sulfonate, - sulfate bzw. -carboxylate nach Abkühlen des Gemischs abgetrennt werden.

2. Verfahren nach Anspruch 1, wobei A Chlorid, Bromid, lodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methylsulfat, Phosphonat, Methylphosphonat, Methandisulfonat, Methylsulfonat, Ethansulfonat bedeutet.

3. Verfahren nach den Ansprüchen 1 bis 2, **dadurch gekennzeichnet, dass** die Umsetzung der Ammoniumnitrile gemäß der Formel 1 mit einem Alkali- oder Ammoniumsalz eines p-Toluolsulfonates, Dodecylbenzolsulfonates, C₈-C₁₈₋Ethersulfates, Natriumsulfat und/oder C₈-C₁₈-Alkylcarboxylates erfolgt.

4. Verfahren nach den Ansprüchen 1 bis 3, **dadurch gekennzeichnet, dass** die Umsetzung der Ammoniumnitrile gemäß der Formel 1 mit einem Alkali- oder Ammoniumsalz von Cumolsulfonat erfolgt.

5. Verfahren nach den Ansprüchen 1 bis 3, **dadurch gekennzeichnet, dass** die Umsetzung der Ammoniumnitrile gemäß der Formel 1 mit einem Alkali- oder Ammoniumsalz von Laurylsulfat und/oder C₈-C₁₈-Alkoholsulfat erfolgt.

6. Verfahren nach einem oder mehreren der Ansprüche 1 bis 5, worin die Verbindungen der Formel 1 in Wasser gelöst sind, und mit einer wässrigen Lösung einer Verbindung der Formeln RSO₃X, R¹SO₄X oder R²COOX bei einem Molverhältnis der Verbindungen der Formeln RSO₃X, R¹SO₄X und R²COOX und der Ammoniumnitrile der Formel 1 im Bereich von 1 zu 2 bis 5 zu 1 bevorzugt 1 zu 1 bis 4 zu 1 umgesetzt werden.

7. Verfahren nach den Ansprüchen 1 bis 5, **dadurch gekennzeichnet, dass** man nach Beendigung der Reaktion die wässrige Lösung oder Dispersion durch Trocknen, insbesondere durch eine Sprühtrocknung, aufarbeitet.

8. Verfahren nach Anspruch 1 bis 7, **dadurch gekennzeichnet, dass** die erhaltenen hydrolysestabilen Ammoniumnitrile granuliert und mit einer Coatingsubstanz überzogen werden.

## Claims

1. A method for producing hydrolysis-stable ammonium nitriles by reacting a compound of the formula 1 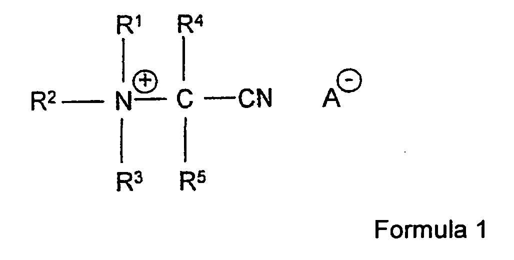 in which R¹, R², R³ are identical or different and are linear or branched C₁-C₂₄-alkyl groups, C₂-C₂₄-alkenyl groups or are C₁-C₄₋alkoxy-C₁-C₄-alkyl groups, substituted or unsubstituted benzyl, or in which R¹ and R² together with the nitrogen atom to which they are bonded form a ring having 4 to 6 carbon atoms, which may be substituted by C₁-C₅-alkyl, C₁-C₅-alkoxy, C₁-C₅-alkanoyl, phenyl, amino, ammonium, cyano, cyanamino, chlorine or bromine and which can contain in addition to the nitrogen atom, instead of carbon atoms, one or two oxygen or nitrogen atoms, a group N-R⁶ or a group R³-N-R⁶, in which R⁶ is hydrogen, C₁- to C₅-alkyl, C₂- to C₅₋alkenyl, C₂-C₅-alkynyl, phenyl, C₇-C₉-aralkyl, C₅-C₇-cycloalkyl, C₁₋C₆-alkanoyl, cyanomethyl or cyano, R⁴ and R⁵ are hydrogen, C₁-C₄₋alkyl, C₁-C₄-alkenyl, C₁-C₄-alkoxy-C₁-C₄-alkyl, phenyl or C₁-C₃₋alkylphenyl, preferably hydrogen, methyl or phenyl, where in particular R⁴ is hydrogen if R⁵ is not hydrogen, and A is an anion, with a compound of the formulae RSO₃X, R¹SO₄X or R²CO₂X, where R, R¹ and R² are C₄-C₂₀-alkyl, preferably C₁₀-C₁₈-alkyl or substituted aryl, preferably C₁-C₁₈-alkylphenyl, and X is an alkali metal ion, ammonium ion or hydrogen ion, where the nitriles of the formula 1 are dissolved in water at 50 to 100°C upto saturation, mixed with an aqueous solution, saturated in the same temperature range, of compounds of the formulae RSO₃X, R¹SO₄X or R²CO₂X, and the ammonium nitrile sulfonates, sulfates or carboxylates are separated off after cooling the mixture.

2. The method as claimed in claim 1, where A is chloride, bromide, iodide, fluoride, sulfate, hydrogen sulfate, carbonate, hydrogen carbonate, phosphate, mono- and dihydrogen phosphate, pyrophosphate, metaphosphate, nitrate, methylsulfate, phosphonate, methylphosphonate, methanedisulfonate, methylsulfonate or ethanesulfonate.

3. The method as claimed in claims 1 to 2, wherein the reaction of the ammonium nitriles according to formula 1 takes place with an alkali metal salt or ammonium salt of a p-toluenesulfonate, dodecylbenzenesulfonate, C₈-C₁₈-ether sulfate, sodium sulfate and/or C₈-C₁₈-alkylcarboxylate.

4. The method as claimed in claims 1 to 3, wherein the reaction of the ammonium nitriles according to formula 1 takes place with an alkali metal salt or ammonium salt of cumenesulfonate.

5. The method as claimed in claims 1 to 3, wherein the reaction of the ammonium nitriles according to formula 1 takes place with an alkali metal salt or ammonium salt of lauryl sulfate and/or C₈-C₁₈-alcohol sulfate.

6. The method as claimed in one or more of claims 1 to 5, in which the compounds of the formula 1 are dissolved in water, and are reacted with an aqueous solution of a compound of the formulae RSO₃X, R¹SO₄X or R²COOX in a molar ratio of the compounds of the formulae RSO₃X, R¹SO₄X or R²COOX and of the ammonium nitriles of the formula 1 in the range from 1:2 to 5:1, preferably 1:1 to 4:1.

7. The method as claimed in claims 1 to 5, wherein, when the reaction is complete, the aqueous solution or dispersion is worked up by drying, in particular by spray-drying.

8. The method as claimed in claims 1 to 7, wherein the hydrolysis-stable ammonium nitriles obtained are granulated and covered with a coating substance.

## Revendications

1. Procédé de préparation de nitriles d'ammonium stables à l'hydrolyse en faisant réagir un composé de formule 1 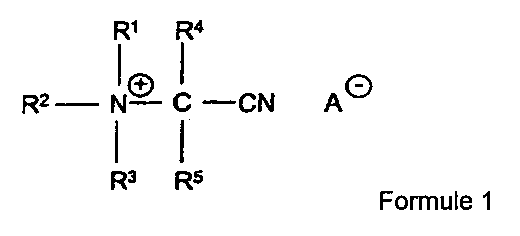 dans laquelle R¹, R², R³ sont identiques ou différents et représentent des groupes alkyle en C₁ à C₂₄ linéaires ou ramifiés, alcényle en C₂ à C₂₄ ou (alcoxy en C₁ à C₄)-(alkyle en C₁ à C₄), un groupe benzyle substitué ou non substitué, ou dans laquelle R¹ et R² forment, conjointement avec l'atome d'azote auquel ils sont liés, un cycle de 4 à 6 atomes de carbone, qui peut être substitué par un groupe alkyle en C₁ à C₅, alcoxy en C₁ à C₅, alcanoyle en C₁ à C₅, phényle, amino, ammonium, cyano, cyanamino, chloro ou bromo et qui peut contenir, en plus de l'atome d'azote, à la place des atomes de carbone, un ou deux atomes d'oxygène ou d'azote, un groupe N-R⁶ ou un groupe R³-NR⁶, dans lequel R⁶ représente un atome d'hydrogène, un groupe alkyle en C₁ à C₅, alcényle en C₂ à C₅, alcynyle en C₂ à C₅, phényle, aralkyle en C₇ à C₉, cycloalkyle en C₅ à C₇, alcanoyle en C₁ à C₆, cyanométhyle ou cyano, R⁴ et R⁵ représentent chacun un atome d'hydrogène, un groupe alkyle en C₁ à C₄, alcényle en C₁ à C₄, (alcoxy en C₁ à C₄)-(alkyle en C₁ à C₄), phényle ou alkyl(en C₁ à C₃)phényle, de préférence un atome d'hydrogène, un groupe méthyle ou phényle, R⁴ représentant en particulier un atome d'hydrogène lorsque R⁵ ne représente pas un atome d'hydrogène, et A représente un anion, avec un composé de formule RSO₃X, R¹SO₄X ou R²CO₂X, dans laquelle R, R¹ et R² représentent chacun un groupe alkyle en C₄ à C₂₀, de préférence en C₁₀ à C₁₈ ou un groupe aryle substitué, de préférence un groupe alkyl (en C₁ à C₁₈)phényle, et X représente un ion alcalin, ammonium ou hydrogène, dans lequel les nitriles de formule 1 sont dissous dans de l'eau à une température de 50°C à 100°C jusqu'à saturation, sont mélangés avec une solution aqueuse saturée des composés de formule RSO₃X, R¹SO₄X ou R²CO₂X dans la même gamme de température, et les sulfonates, sulfates ou carboxylates de nitrile d'ammonium sont séparés après refroidissement du mélange.

2. Procédé selon la revendication 1, dans lequel A représente un chlorure, un bromure, un iodure, un fluorure, un sulfate, un hydrogénosulfate, un carbonate, un hydrogénocarbonate, un phosphate, un mono- et un di-hydrogénophosphate, un pyrophosphate, un métaphosphate, un nitrate, un méthylsulfate, un phosphonate, un méthylphosphonate, un méthanedisulfonate, un méthylsulfonate, un éthanesulfonate.

3. Procédé selon les revendications 1 à 2, **caractérisé en ce que** la réaction des nitriles d'ammonium de formule 1 est réalisée avec un sel alcalin ou d'ammonium d'un p-toluènesulfonate, d'un dodécylbenzènesulfonate, d'un éthersulfate en C₈ à C₁₈, d'un sulfate de sodium et/ou d'un alkyl(en C₈ à C₁₈)carboxylate.

4. Procédé selon les revendications 1 à 3, **caractérisé en ce que** la réaction des nitriles d'ammonium de formule 1 est réalisée avec un sel alcalin ou d'ammonium de cumènesulfonate.

5. Procédé selon les revendications 1 à 3, **caractérisé en ce que** la réaction des nitriles d'ammonium de formule 1 est réalisée avec un sel alcalin ou d'ammonium de laurylsulfate et/ou d'un alcool(en C₈ à C₁₈) sulfate.

6. Procédé selon l'une quelconque ou plusieurs des revendications 1 à 5, dans lequel les composés de formule 1 sont dissous dans l'eau et sont mis à réagir avec une solution aqueuse d'un composé de formule RSO₃X, R¹SO₄X ou R²COOX, avec un rapport molaire des composés de formules RSO₃X, R¹SO₄X et R²COOX aux nitriles d'ammonium de formule 1 dans la gamme de 1:2 à 5:1, de préférence de 1:1 à 4:1.

7. Procédé selon les revendications 1 à 5, **caractérisé en ce que**, une fois la réaction terminée, on soumet la solution ou la dispersion aqueuse à un séchage, en particulier à un séchage par pulvérisation.

8. Procédé selon les revendications 1 à 7, **caractérisé en ce que** l'on granule les nitriles d'ammonium stables à l'hydrolyse obtenus et on les enrobe d'une substance de revêtement.
